# EUROPEAN PATENT SPECIFICATION

(11) **EP 1 761 273 B1**
(45) Date of publication and mention of the grant of the patent: **14.05.2008**
(21) Application number: 05753069.3
(22) Date of filing: 20.06.2005
(51) Int. Cl.: A61K 38/46, A61K 48/00, A61P 1/16

(54) **TREATMENT OF NON ALCOHOLIC STEATOTIC HEPATITIS (NASH)**
BEHANDLUNG VON NICHT-ALKOHOLISCHER STEATOTISCHER HEPATITIS (NASH)
TRAITEMENT DE L'HEPATITE STEATOSIQUE NON ALCOOLIQUE (NASH)

(30) Priority: 21.06.2004 US 580903 P
(43) Date of publication of application: 14.03.2007
(73) Proprietor: Amsterdam Molecular Therapeutics B.V., 1105 BA Amsterdam (NL)
(72) Inventor: VAN DEVENTER, Sander, Jan, Hendrik, NL-2012 CH Haarlem (NL)
(74) Representative: Swinkels, Bart Willem
(86) International application number: PCT/NL2005/000446
(87) International publication number: WO 2005/123117

(56) References cited:
- WO-A-01/00220
- "Correction of Dyslipidemia in Murine and Feline Models of Lipoprotein Lipase Deficiency by Intramuscular Administration of AAV1-LPL<S447X>" MOLECULAR THERAPY, ACADEMIC PRESS, SAN DIEGO, CA,, US, vol. 9, May 2004 (2004-05), page 17, XP004634406 ISSN: 1525-0016
- ROSS COLIN J D ET AL: "Long-term efficacy of a single administration of AAV lipoprotein lipase gene therapy corrects murine hypertriglyceridemia and raises HDL." CIRCULATION, vol. 106, no. 19 Supplement, 5 November 2002 (2002-11-05), pages II-126, XP009052521 & ABSTRACTS FROM SCIENTIFIC SESSIONS; CHICAGO, IL, USA; NOVEMBER 17-20, 2002 ISSN: 0009-7322
- DEN BOER M ET AL: "Hepatic steatosis: A mediator of the metabolic syndrome. Lessons from animal models" ARTERIOSCLEROSIS THROMBOSIS AND VASCULAR BIOLOGY, VOL. 24, NO. 4, PP. 644-649. ISSN: 1079-5642. PB - LIPPINCOTT WILLIAMS & WILKINS, 530 WALNUT ST, PHILADELPHIA, PA 19106-3621 USA., April 2004 (2004-04), XP009052482
- FEHER J ET AL: "A NEW APPROACH TO DRUG THERAPY IN NON-ALCOHOLIC STEATOHEPATITIS (NASH)" JOURNAL OF INTERNATIONAL MEDICAL RESEARCH, CAMBRIDGE MEDICAL PUBLICATIONS LTD, GB, vol. 31, no. 6, 2003, pages 537-551, XP008043716 ISSN: 0300-0605

### SEQUENCE LISTING

<110> Amsterdams Medisch Centrum and Amsterdam Molecular Therapeutics
<120> novel compounds for treatment of nonalcoholic steatotic hepatitis
<130> P215797
<160> 2
<170> PatentIn version 3.3
<210> 1
   <211> 446
   <212> PRT
   <213> Homo sapiens
<400> 1
   Ala Asp Gln Arg Arg Asp Phe Ile Asp Ile Glu Ser Lys Phe Ala Leu
   1               5                   10                  15
   Arg Thr Pro Glu Asp Thr Ala Glu Asp Thr Cys His Leu Ile Pro Gly
               20                  25                  30
   Val Ala Glu Ser Val Ala Thr Cys His Phe Asn His Ser Ser Lys Thr
           35                  40                  45
   Phe Met Val Ile His Gly Trp Thr Val Thr Gly Met Tyr Glu Ser Trp
       50                  55                  60
   Val Pro Lys Leu Val Ala Ala Leu Tyr Lys Arg Glu Pro Asp Ser Asn
   65                  70                  75                  80
   Val Ile Val Val Asp Trp Leu Ser Arg Ala Gln Glu His Tyr Pro Val
                   85                  90                  95
   Ser Ala Gly Tyr Thr Lys Leu Val Gly Gln Asp Val Ala Arg Phe Ile
               100                 105                 110
   Asn Trp Met Glu Glu Glu Phe Asn Tyr Pro Leu Asp Asn Val His Leu
           115                 120                 125
   Leu Gly Tyr Ser Leu Gly Ala His Ala Ala Gly Ile Ala Gly Ser Leu
       130                 135                 140
   Thr Asn Lys Lys Val Asn Arg Ile Thr Gly Leu Asp Pro Ala Gly Pro
   145                 150                 155                 160
   Asn Phe Glu Tyr Ala Glu Ala Pro Ser Arg Leu Ser Pro Asp Asp Ala
                   165                 170                 175
   Asp Phe Val Asp Val Leu His Thr Phe Thr Arg Gly Ser Pro Gly Arg
               180                 185                 190
   Ser Ile Gly Ile Gln Lys Pro Val Gly His Val Asp Ile Tyr Pro Asn
   195 200 205
   Gly Gly Thr Phe Gln Pro Gly Cys Asn Ile Gly Glu Ala Ile Arg Val
   210 215 220
   Ile Ala Glu Arg Gly Leu Gly Asp Val Asp Gln Leu Val Lys Cys Ser
   225 230 235 240
   His Glu Arg Ser Ile His Leu Phe Ile Asp Ser Leu Leu Asn Glu Glu
   245 250 255
   Asn Pro Ser Lys Ala Tyr Arg Cys Ser Ser Lys Glu Ala Phe Glu Lys
   260 265 270
   Gly Leu Cys Leu Ser Cys Arg Lys Asn Arg Cys Asn Asn Leu Gly Tyr
   275 280 285
   Glu Ile Asn Lys Val Arg Ala Lys Arg Ser Ser Lys Met Tyr Leu Lys
   290 295 300
   Thr Arg Ser Gln Met Pro Tyr Lys Val Phe His Tyr Gln Val Lys Ile
   305 310 315 320
   His Phe Ser Gly Thr Glu Ser Glu Thr His Thr Asn Gln Ala Phe Glu
   325 330 335
   Ile Ser Leu Tyr Gly Thr Val Ala Glu Ser Glu Asn Ile Pro Phe Thr
   340 345 350
   Leu Pro Glu Val Ser Thr Asn Lys Thr Tyr Ser Phe Leu Ile Tyr Thr
   355 360 365
   Glu Val Asp Ile Gly Glu Leu Leu Met Leu Lys Leu Lys Trp Lys Ser
   370 375 380
   Asp Ser Tyr Phe Ser Trp Ser Asp Trp Trp Ser Ser Pro Gly Phe Ala
   385 390 395 400
   Ile Gln Lys Ile Arg Val Lys Ala Gly Glu Thr Gln Lys Lys Val Ile
   405 410 415
   Phe Cys Ser Arg Glu Lys Val Ser His Leu Gln Lys Gly Lys Ala Pro
   420 425 430
   Ala Val Phe Val Lys Cys His Asp Lys Ser Leu Asn Lys Lys
   435 440 445
<210> 2
   <211> 3549
   <212> DNA
   <213> Homo sapiens
<400> 2
   cccctcttcc tcctcctcaa gggaaagctg cccacttcta gctgccctgc catccccttt     60
   aaagggcgac ttgctcagcg ccaaaccgcg gctccagccc tctccagcct ccggctcagc    120
   cggctcatca gtcggtccgc gccttgcagc tcctccagag ggacgcgccc cgagatggag    180
   agcaaagccc tgctcgtgct gactctggcc gtgtggctcc agagtctgac cgcctcccgc    240
   ggaggggtgg ccgccgccga ccaaagaaga gattttatcg acatcgaaag taaatttgcc    300
   ctaaggaccc ctgaagacac agctgaggac acttgccacc tcattcccgg agtagcagag    360
   tccgtggcta cctgtcattt caatcacagc agcaaaacct tcatggtgat ccatggctgg    420
   acggtaacag gaatgtatga gagttgggtg ccaaaacttg tggccgccct gtacaagaga    480
   gaaccagact ccaatgtcat tgtggtggac tggctgtcac gggctcagga gcattaccca    540
   gtgtccgcgg gctacaccaa actggtggga caggatgtgg cccggtttat caactggatg    600
   gaggaggagt ttaactaccc tctggacaat gtccatctct tgggatacag ccttggagcc    660
   catgctgctg gcattgcagg aagtctgacc aataagaaag tcaacagaat tactggcctc    720
   gatccagctg gacctaactt tgagtatgca gaagccccga gtcgtctttc tcctgatgat    780
   gcagattttg tagacgtctt acacacattc accagagggt cccctggtcg aagcattgga    840
   atccagaaac cagttgggca tgttgacatt tacccgaatg gaggtacttt tcagccagga    900
   tgtaacattg gagaagctat ccgcgtgatt gcagagagag gacttggaga tgtggaccag    960
   ctagtgaagt gctcccacga gcgctccatt catctcttca tcgactctct gttgaatgaa   1020
   gaaaatccaa gtaaggccta caggtgcagt tccaaggaag cctttgagaa agggctctgc   1080
   ttgagttgta gaaagaaccg ctgcaacaat ctgggctatg agatcaataa agtcagagcc   1140
   aaaagaagca gcaaaatgta cctgaagact cgttctcaga tgccctacaa agtcttccat   1200
   taccaagtaa agattcattt ttctgggact gagagtgaaa cccataccaa tcaggccttt   1260
   gagatttctc tgtatggcac cgtggccgag agtgagaaca tcccattcac tctgcctgaa   1320
   gtttccacaa ataagaccta ctccttccta atttacacag aggtagatat tggagaacta   1380
   ctcatgttga agctcaaatg gaagagtgat tcatacttta gctggtcaga ctggtggagc   1440
   agtcccggct tcgccattca gaagatcaga gtaaaagcag gagagactca gaaaaaggtg   1500
   atcttctgtt ctagggagaa agtgtctcat ttgcagaaag gaaaggcacc tgcggtattt   1560
   gtgaaatgcc atgacaagtc tctgaataag aagtcaggct gaaactgggc gaatctacag   1620
   aacaaagaac ggcatgtgaa ttctgtgaag aatgaagtgg aggaagtaac ttttacaaaa   1680
   catacccagt gtttggggtg tttcaaaagt ggattttcct gaatattaat cccagcccta   1740
   cccttgttag ttattttagg agacagtctc aagcactaaa aagtggctaa ttcaatttat   1800
   ggggtatagt ggccaaatag cacatcctcc aacgttaaaa gacagtggat catgaaaagt   1860
   gctgttttgt cctttgagaa agaaataatt gtttgagcgc agagtaaaat aaggctcctt   1920
   catgtggcgt attgggccat agcctataat tggttagaac ctcctatttt aattggaatt   1980
   ctggatcttt cggactgagg ccttctcaaa ctttactcta agtctccaag aatacagaaa 2040
   atgcttttcc gcggcacgaa tcagactcat ctacacagca gtatgaatga tgttttagaa 2100
   tgattccctc ttgctattgg aatgtggtcc agacgtcaac caggaacatg taacttggag 2160
   agggacgaag aaagggtctg ataaacacag aggttttaaa cagtccctac cattggcctg 2220
   catcatgaca aagttacaaa ttcaaggaga tataaaatct agatcaatta attcttaata 2280
   ggctttatcg tttattgctt aatccctctc tcccccttct tttttgtctc aagattatat 2340
   tataataatg ttctctgggt aggtgttgaa aatgagcctg taatcctcag ctgacacata 2400
   atttgaatgg tgcagaaaaa aaaaagatac cgtaatttta ttattagatt ctccaaatga 2460
   ttttcatcaa tttaaaatca ttcaatatct gacagttact cttcagtttt aggcttacct 2520
   tggtcatgct tcagttgtac ttccagtgcg tctcttttgt tcctggcttt gacatgaaaa 2580
   gataggtttg agttcaaatt ttgcattgtg tgagcttcta cagattttag acaaggaccg 2640
   tttttactaa gtaaaagggt ggagaggttc ctggggtgga ttcctaagca gtgcttgtaa 2700
   accatcgcgt gcaatgagcc agatggagta ccatgagggt tgttatttgt tgtttttaac 2760
   aactaatcaa gagtgagtga acaactattt ataaactaga tctcctattt ttcagaatgc 2820
   tcttctacgt ataaatatga aatgataaag atgtcaaata tctcagaggc tatagctggg 2880
   aacccgactg tgaaagtatg tgatatctga acacatacta gaaagctctg catgtgtgtt 2940
   gtccttcagc ataattcgga agggaaaaca gtcgatcaag ggatgtattg gaacatgtcg 3000
   gagtagaaat tgttcctgat gtgccagaac ttcgaccctt tctctgagag agatgatcgt 3060
   gcctataaat agtaggacca atgttgtgat taacatcatc aggcttggaa tgaattctct 3120
   ctaaaaataa aatgatgtat gatttgttgt tggcatcccc tttattaatt cattaaattt 3180
   ctggatttgg gttgtgaccc agggtgcatt aacttaaaag attcactaaa gcagcacata 3240
   gcactgggaa ctctggctcc gaaaaacttt gttatatata tcaaggatgt tctggcttta 3300
   cattttattt attagctgta aatacatgtg tggatgtgta aatggagctt gtacatattg 3360
   gaaaggtcat tgtggctatc tgcatttata aatgtgtggt gctaactgta tgtgtcttta 3420
   tcagtgatgg tctcacagag ccaactcact cttatgaaat gggctttaac aaaacaagaa 3480
   agaaacgtac ttaactgtgt gaagaaatgg aatcagcttt taataaaatt gacaacattt 3540
   tattaccac 3549

## Description

### Field of the invention

The present invention is in the field of protein and nucleic acid therapeutics for the treatment of non-alcoholic steatotic hepatitis.

### Background of the invention

Non-alcoholic steatotic hepatitis (NASH) is part of a spectrum of nonalcoholic fatty liver disease (NAFL) and refers to the development of histological changes in the liver that are comparable to those induced by excessive alcohol intake. However NASH occurs in patients who are not abusing alcohol. NASH is characterized by elevated serum aminotransferases, indicating liver cell damage. Macrovesicular steatotis (i.e, intracytoplasmic vacuoles that eccentrically displace the hepatocyte nucleus), inflammation, and occasionally fibrosis that may progress to cirrhosis, characterise the disease. There is increasing evidence that NASH is a component of the metabolic insulin resistance syndrome. This is a cluster of disorders, including obesity, dyslipidemia, arteriosclerosis and diabetes mellitis, which have insulin resistance as a common feature (de Sligte et al (2004) Eur J Int Med 15:10).

Although many drugs have been tried to improve NASH and prevent further detorioration, there is no established treatment for this potentially serious condition. One of the drugs tested for efficacy in NASH is rosiglitazone (Muurling et al (2003) Metabolism 52: 1078). A loss of body weight may also improve NASH. However, despite these interventions, in a significant number of patient NASH progresses to cirrhosis and it may ultimately require liver transplantation, after which the disease may recur.

Given the trend of increasing obesity and diabetes in both the U.S. and the European populations, there is an urgent need for novel treatments of NASH.

### Detailed description

The present invention provides a method of treating non alcoholic steatotic hepatitis in a subject. The method comprises administering to a subject an effective amount of a lipoprotein lipase (LPL) therapeutic. As used herein, LPL therapeutic refers to a protein with LPL activity (EC 3.1.1.34) or to a nucleic acid encoding such a protein.

### The protein form of LPL therapeutic

In one embodiment, the LPL therapeutic is an LPL protein with an amino acid sequence as shown in SEQ ID No. 1, herein referred to as LPL S447X proteins or peptides. In general, these LPL S447X proteins are shorter than well-known wild type LPL, which has 448 amino acids (see for instance Wion et al Science (1987) 235: 1638 or WO 01/00220). They may include compounds such as peptide fragments, modified peptide fragments, analogues or pharmacologically acceptable salts of LPL having amino acids 447-448 truncated from the carboxy terminal of a wild-type LPL. Such compounds are collectively referred to herein as LPL S447X peptides. LPL S447X peptides may include homologs of the wild-type mature LPL sequence from amino acids 1 through 446, including homologs from species other than *homo sapiens* (which may have veterinary applications). LPL S447X peptides may include derivatives and naturally occurring isoforms or genetic variants of wild type LPL. The use of derivatives and variants of the LPL S447X protein is also encompassed in the invention.

### Derivatives of S447X protein

Derivatives include, in particular, proteins with LPL activity which have the same amino acid sequence as LPL S447X protein, but in which some N- or O-glycosylation sites have been modified or eliminated. Derivatives also include C-terminal hydroxymethyl derivatives, O-modified derivatives (e.g., C-terminal hydroxymethyl benzyl ether), and N-terminally modified derivatives including substituted amides such as alkylamides and hydrazides.

Within an LPL therapeutic of the invention, a peptidic structure maybe coupled directly or indirectly to a modifying group. The term "modifying group" is intended to include structures that are directly attached to the peptidic structure (e.g., by covalent coupling), as well as those that are indirectly attached to the peptidic structure (e.g., by a stable non-covalent association or by covalent coupling to additional amino acid residues, or mimetics, analogues or derivatives thereof, which may flank the MCP-3 core peptidic structure). For example, the modifying group can be coupled to the amino-terminus or carboxy-terminus of an LPL therapeutic structure, or to a peptidic or peptidomimetic region flanking the core domain. Alternatively, the modifying group can be coupled to a side chain of an amino acid residue of the LPL therapeutic, or to a peptidic or peptido-mimetic region flanking the core domain (e.g., through the epsilon amino group of a lysyl residue(s), through the carboxyl group of an aspartic acid residue(s) or a glutamic acid residue(s), through a hydroxy group of a tyrosyl residue(s), a serine residue(s) or a threonine residue(s) or other suitable reactive group on an amino acid side chain). Modifying groups covalently coupled to the peptidic structure can be attached by means and using methods well known in the art for linking chemical structures, including, for example, amide, alkylamino, carbamate or urea bonds.

In some embodiments, the modifying group may comprise a cyclic, heterocyclic or polycyclic group. The term "cyclic group", as used herein, includes cyclic saturated or unsaturated (i.e., aromatic) group having from 3 to 10, 4 to 8, or 5 to 7 carbon atoms. Exemplary cyclic groups include cyclopropyl, cyclobutyl, cyclopentyl, cyclohexyl, and cyclooctyl. Cyclic groups may be unsubstituted or substituted at one or more ring positions. A cyclic group may for example be substituted with halogens, alkyls, cycloalkyls, alkenyls, alkynyls, aryls, heterocycles, hydroxyls, aminos, nitros, thiols amines, imines, amides, phosphonates, phosphines, carbonyls, carboxyls, silyls, ethers, thioethers, sulfonyls, sulfonates, selenoethers, ketones, aldehydes, esters, -CF₃, -CN.

The term "heterocyclic group" includes cyclic saturated, unsaturated and aromatic groups having from 3 to 10, 4 to 8, or 5 to 7 carbon atoms, wherein the ring structure includes about one or more heteroatoms. Heterocyclic groups include pyrrolidine, oxolane, thiolane, imidazole, oxazole, piperidine, piperazine, morpholine. The heterocyclic ring may be substituted at one or more positions with such substituents as, for example, halogens, alkyls, cycloalkyls, alkenyls, alkynyls, aryls, other heterocycles, hydroxyl, amino, nitro, thiol, amines, imines, amides, phosphonates, phosphines, carbonyls, carboxyls, silyls, ethers, thioethers, sulfonyls, selenoethers, ketones, aldehydes, esters, -CF₃, -CN. Heterocycles may also be bridged or fused to other cyclic groups as described below.

The term "polycyclic group" as used herein is intended to refer to two or more saturated, unsaturated or aromatic cyclic rings in which two or more carbons are common to two adjoining rings, so that the rings are "fused rings". Rings that are joined through non-adjacent atoms are termed "bridged" rings. Each of the rings of the polycyclic group may be substituted with such substituents as described above, as for example, halogens, alkyls, cycloalkyls, alkenyls, alkynyls, hydroxyl, amino, nitro, thiol, amines, imines, amides, phosphonates, phosphines, carbonyls, carboxyls, silyls, ethers, thioethers, sulfonyls, selenoethers, ketones, aldehydes, esters, -CF₃, or -CN.

The term "alkyl" refers to the radical of saturated aliphatic groups, including straight chain alkyl groups, branched-chain alkyl groups, cycloalkyl (alicyclic) groups, alkyl substituted cycloalkyl groups, and cycloalkyl substituted alkyl groups. In some embodiments, a straight chain or branched chain alkyl has 20 or fewer carbon atoms in its backbone (C₁-C₂₀ for straight chain, C₃-C₂₀ for branched chain), or 10 or fewer carbon atoms . In some embodiments, cycloalkyls may have from 4-10 carbon atoms in their ring structure, such as 5, 6 or 7 carbon rings. Unless the number of carbons is otherwise specified, "lower alkyl" as used herein means an alkyl group, as defined above, having from one to ten carbon atoms in its backbone structure. Likewise, "lower alkenyl" and "lower alkynyl" have chain lengths of ten or less carbons.

The term "alkyl" (or "lower alkyl") as used throughout the specification and claims is intended to include both "unsubstituted alkyls" and "substituted alkyls", the latter of which refers to alkyl moieties having substituents replacing a hydrogen on one or more carbons of the hydrocarbon backbone. Such substituents can include, for example, halogen, hydroxyl, carbonyl (such as carboxyl, ketones (including alkylcarbonyl and arylcarbonyl groups), and esters (including alkyloxycarbonyl and aryloxycarbonyl groups)), thiocarbonyl, acyloxy, alkoxyl, phosphoryl, phosphonate, phosphinate, amino, acylamino, amido, amidine, imino, cyano, nitro, azido, sulfhydryl, alkylthio, sulfate, sulfonate, sulfamoyl, sulfonamido, heterocyclyl, aralkyl, or an aromatic or heteroaromatic moiety. The moieties substituted on the hydrocarbon chain can themselves be substituted, if appropriate. For instance, the substituents of a substituted alkyl may include substituted and unsubstituted forms of aminos, azidos, iminos, amidos, phosphoryls (including phosphonates and phosphinates), sulfonyls (including sulfates, sulfonamidos, sulfamoyls and sulfonates), and silyl groups, as well as ethers, alkylthios, carbonyls (including ketones, aldehydes, carboxylates, and esters), -CF₃, - CN and the like. Exemplary substituted alkyls are described below. Cycloalkyls can be further substituted with alkyls, alkenyls, alkoxys, alkylthios, aminoalkyls, carbonyl-substituted alkyls, -CF₃, -CN, and the like.

The terms "alkenyl" and "alkynyl" refer to unsaturated aliphatic groups analogous in length and possible substitution to the alkyls described above, but that contain at least one double or triple bond respectively.

The term "aralkyl", as used herein, refers to an alkyl or alkylenyl group substituted with at least one aryl group. Exemplary aralkyls include benzyl (i.e., phenylmethyl), 2-naphthylethyl, 2-(2-pyridyl)propyl, 5-dibenzosuberyl, and the like.

The term "alkylcarbonyl", as used herein, refers to -C(O)-alkyl. Similarly, the term "arylcarbonyl" refers to -C(O)-aryl. The term "alkyloxycarbonyl", as used herein, refers to the group -C(O)-O-alkyl, and the term "aryloxycarbonyl" refers to =C(O)-O-aryl. The term "acyloxy" refers to -O-C(O)-R₇, in which R₇ is alkyl, alkenyl, alkynyl, aryl, aralkyl or heterocyclyl.

The term "amino", as used herein, refers to -N(R_{α})(R_{β}), in which R_{α} and R_{β} are each independently hydrogen, alkyl, alkyenyl, alkynyl, aralkyl, aryl, or in which R_{α} and R_{β} together with the nitrogen atom to which they are attached form a ring having 4-8 atoms. Thus, the term "amino", as used herein, includes unsubstituted, monosubstituted (e.g., monoalkylamino or monoarylamino), and disubstituted (e.g., dialkylamino or alkylarylamino) amino groups. The term "amido" refers to -C(O)-N(R₈)(R₉), in which R₈ and R₉ are as defined above. The term "acylamino" refers to -N(R'₈)C(O)-R₇, in which R₇ is as defined above and R'₈ is alkyl.

As used herein, the term "nitro" means -NO₂ ; the term "halogen" designates -F, -Cl, -Br or -I; the term "sulfhydryl" means -SH; and the term "hydroxyl" means -OH.

The term "aryl" as used herein includes 5-, 6- and 7-membered aromatic groups that may include from zero to four heteroatoms in the ring, for example, phenyl, pyrrolyl, furyl, thiophenyl, imidazolyl, oxazole, thiazolyl, triazolyl, pyrazolyl, pyridyl, pyrazinyl, pyridazinyl and pyrimidinyl, and the like. Those aryl groups having heteroatoms in the ring structure may also be referred to as "aryl heterocycles" or "heteroaromatics". The aromatic ring can be substituted at one or more ring positions with such substituents as described above, as for example, halogen, azide, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, sulfonamido, ketone, aldehyde, ester, a heterocyclyl, an aromatic or heteroaromatic moiety, -CF₃, -CN, or the like. Aryl groups can also be part of a polycyclic group. For example, aryl groups include fused aromatic moieties such as naphthyl, anthracenyl, quinolyl, indolyl, and the like.

Modifying groups may include groups comprising biotinyl structures, fluorescein-containing groups, a diethylene-triaminepentaacetyl group, a (-)-menthoxyacetyl group, a N-acetylneuraminyl group, a cholyl structure or an iminiobiotinyl group. An LPL therapeutic may be modified at its carboxy terminus with a cholyl group according to methods known in the art (for example see: Wess, G. et al. (1993) Tetrahedron Letters, 34:817-822; Cholyl derivatives and analogues may also be used as modifying groups, such as Aic (3-(O-aminoethyl-iso)-cholyl), which has a free amino group that can be used to further modify the LPL therapeutic. A modifying group may be a "biotinyl structure", which includes biotinyl groups and analogues and derivatives thereof (such as a 2-iminobiotinyl group). In another embodiment, the modifying group may comprise a "fluorescein-containing group", such as a group derived from reacting an LPL therapeutic peptide with 5-(and 6-)-carboxyfluorescein, succinimidyl ester or fluorescein isothiocyanate. In various other embodiments, the modifying group(s) may comprise an N-acetylneuraminyl group, a trans-4-cotininecarboxyl group, a 2-imino-1-imidazolidineacetyl group, an (S)-(-)-indoline-2-carboxyl group, a (-)-menthoxyacetyl group, a 2-norbornaneacetyl group, a gamma-oxo-5-acenaphthenebutyryl, a (-)-2-oxo-4-thiazolidinecarboxyl group, a tetrahydro-3-furoyl group, a 2-iminobiotinyl group, a diethylenetriaminepentaacetyl group, a 4-morpholinecarbonyl group, a 2-thiopheneacetyl group or a 2-thiophenesulfonyl group.

### Variants of LPL S447X protein

Variants LPL polypeptides include polypeptides having substantial sequence similarity to LPL S447X protein, such as 90%, 95% or 99% sequence identity to a corresponding portion of SEQ ID No. 1, the corresponding portion being any contiguous sequence of any length, such as 10, 20, 30, 40, 50 or more amino acids. Such proteins typically have LPL activity, or another LPL-like property, equal to or greater than LPL S447X protein. In some embodiments, chemically similar amino acids may be substituted for amino acids in the LPL S447X protein sequence (to provide conservative amino acid substitutions). Amino acid substitutions that reduce LPL activity, of which more than 50 have been disclosed, such as the substitution of a Ser residue for Asn at position 291 (Asn291 Ser), the substitution of Asn for Asp at position 9 (Asp9Asn), the substitution of Glu for Gly at position 188 (Gly188Glu, see Monsalve et al,. J.Clin.Invest. 1990, 86(3):728-734) or Asp250Asn (Ma et al., Genomics. 1992, 13:649-653) should be avoided in preferred embodiments.

### The nucleic acid form of LPL therapeutic

In yet another embodiment, the LPL therapeutic is a nucleic acid encoding the wild type LPL protein or encoding a LPL S447X protein. In an alternative embodiment, the nucleic acid may comprise a DNA coding sequence encoding an RNA having at least 90% sequence identity to nucleotides 256 through 1599 of SEQ ID NO:2, which stretch of nucleotides encodes the mature wild type LPL peptide.

Yet alternatively, the LPL therapeutic may comprise a nucleic acid which comprises a DNA coding sequence that hybridizes under stringent conditions to nucleotides 256 through 1599 of SEQ ID NO:2.

### Sequence identity

Two nucleic acid or protein sequences are considered substantially identical if, when optimally aligned, they share at least about 70% sequence identity. In alternative embodiments, sequence identity may for example be at least 75%, at least 90% or at least 95%. Optimal alignment of sequences for comparisons of identity may be conducted using a variety of algorithms, such as the local homology algorithm of Smith and Waterman, 1981, Adv. Appl. Math 2: 482, the homology alignment algorithm of Needleman and Wunsch, 1970, J. Mol. Biol. 48:443, the search for similarity method of Pearson and Lipman, 1988, Proc. Natl. Acad. Sci. USA 85: 2444, and the computerised implementations of these algorithms (such as GAP, BESTFIT, FASTA and TFASTA in the Wisconsin Genetics Software Package, Genetics Computer Group, Madison, WI, U.S.A.). Sequence alignment may also be carried out using the BLAST algorithm, described in Altschul et al., 1990, J. Mol. Biol. 215:403-10 (using the published default settings). Software for performing BLAST analysis may be available through the National Center for Biotechnology Information (through the internet at http://www.ncbi.nlm.nih.gov/). The BLAST algorithm involves first identifying high scoring sequence pairs (HSPs) by identifying short words of length W in the query sequence that either match or satisfy some positive-valued threshold score T when aligned with a word of the same length in a database sequence. T is referred to as the neighbourhood word score threshold. Initial neighbourhood word hits act as seeds for initiating searches to find longer HSPs. The word hits are extended in both directions along each sequence for as far as the cumulative alignment score can be increased. Extension of the word hits in each direction is halted when the following parameters are met: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST programs may use as defaults a word length (W) of 11, the BLOSUM62 scoring matrix (Henikoff and Henikoff, 1992, Proc. Natl. Acad. Sci. USA 89: 10915-10919) alignments (B) of 50, expectation (E) of 10 (which may be changed in alternative embodiments to 1 or 0.1 or 0.01 or 0.001 or 0.0001; although E values much higher than 0.1 may not identify functionally similar sequences, it is useful to examine hits with lower significance, E values between 0.1 and 10, for short regions of similarity), M=5, N=4, for nucleic acids a comparison of both strands. For protein comparisons, BLASTP may be used with defaults as follows: G=11 (cost to open a gap); E=1 (cost to extend a gap); E=10 (expectation value, at this setting, 10 hits with scores equal to or better than the defined alignment score, S, are expected to occur by chance in a database of the same size as the one being searched; the E value can be increased or decreased to alter the stringency of the search.); and W=3 (word size, default is 11 for BLASTN, 3 for other blast programs).

The BLOSUM matrix assigns a probability score for each position in an alignment that is based on the frequency with which that substitution is known to occur among consensus blocks within related proteins. The BLOSUM62 (gap existence cost = 11; per residue gap cost = 1; lambda ratio = 0.85) substitution matrix is used by default in BLAST 2.0. A variety of other matrices may be used as alternatives to BLOSUM62, including: PAM30 (9,1,0.87); PAM70 (10,1,0.87) BLOSUM80 (10,1,0.87); BLOSUM62 (11,1,0.82) and BLOSUM45 (14,2,0.87). One measure of the statistical similarity between two sequences using the BLAST algorithm is the smallest sum probability (P(N)), which provides an indication of the probability by which a match between two nucleotide or amino acid sequences would occur by chance. In alternative embodiments of the invention, nucleotide or amino acid sequences are considered substantially identical if the smallest sum probability in a comparison of the test sequences is less than about 1, preferably less than about 0.1, more preferably less than about 0.01, and most preferably less than about 0.001.

### Modifications

It is well known in the art that some modifications and changes can be made in the structure of a polypeptide without substantially altering the biological function of that peptide, to obtain a biologically equivalent polypeptide. In one aspect of the invention, LPL therapeutics may include peptides that differ from a portion of the wild-type LPL sequence by conservative amino acid substitutions. As used herein, the term "conserved amino acid substitutions" refers to the substitution of one amino acid for another at a given location in the peptide, where the substitution can be made without loss of function. In making such changes, substitutions of like amino acid residues can be made, for example, on the basis of relative similarity of side-chain substituents, for example, their size, charge, hydrophobicity, hydrophilicity, and the like, and such substitutions may be assayed for their effect on the function of the peptide by routine testing.

In some embodiments, conserved amino acid substitutions may be made where an amino acid residue is substituted for another having a similar hydrophilicity value (e.g., within a value of plus or minus 2.0), where the following hydrophilicity values are assigned to amino acid residues (as detailed in United States Patent No. 4,554,101, incorporated herein by reference): Arg (+3.0); Lys (+3.0); Asp (+3.0); Glu (+3.0); Ser (+0.3); Asn (+0.2); Gln (+0.2); Gly (0); Pro (-0.5); Thr (-0.4); Ala (-0.5); His (-0.5); Cys (-1.0); Met (-1.3); Val (-1.5); Leu (-1.8); Ile (-1.8); Tyr (-2.3); Phe (-2.5); and Trp (-3.4).

In alternative embodiments, conserved amino acid substitutions may be made where an amino acid residue is substituted for another having a similar hydropathic index (e.g., within a value of plus or minus 2.0). In such embodiments, each amino acid residue may be assigned a hydropathic index on the basis of its hydrophobicity and charge characteristics, as follows: Ile (+4.5); Val (+4.2); Leu (+3.8); Phe (+2.8); Cys (+2.5); Met (+1.9); Ala (+1.8); Gly (-0.4); Thr (-0.7); Ser (-0.8); Trp (-0.9); Tyr (-1.3); Pro (-1.6); His (-3.2); Glu (-3.5); Gln (-3.5); Asp (-3.5); Asn (-3.5); Lys (-3.9); and Arg (-4.5).

In alternative embodiments, conserved amino acid substitutions may be made where an amino acid residue is substituted for another in the same class, where the amino acids are divided into non-polar, acidic, basic and neutral classes, as follows: non-polar: Ala, Val, Leu, Ile, Phe, Trp, Pro, Met; acidic: Asp, Glu; basic: Lys, Arg, His; neutral: Gly, Ser, Thr, Cys, Asn, Gln, Tyr.

### Gene therapy

In one embodiment of the invention, the LPL therapeutic is administered to a subject in a gene therapy vector. Vectors may be prepared from different type of viruses, including adenoviruses, adeno-associated viruses (AAV), herpes viruses (HSV), lentiviruses and retroviruses. In one embodiment, an AAV serotype selected from the group consisting of: AAV1, AAV2, AAV3, AAV4, AAV5, AAV6, AAV7 and AAV8 is used.

It is within the technical skills of the skilled person to select the most appropriate virus or virus subtype. Some subtypes may be more appropiate than others for a certain type of tissue. For example, muscle-specific over-expression of LPL may advantageously be induced by adeno-associated virus (AAV)-mediated transduction of muscle cells. Muscle is amenable to AAV-mediated transduction, and different serotypes may be used (AAV1, AAV6, AAV7, AAV8). Transduction of muscle is accomplished by intramuscular injection of AAV-LPL in multiple sites. Multiple sites, keeping the local viral dose low, will help to prevent LPL-induced myopathy or vector-induced immune responses. This has been an effective method for long-term transduction of muscle using serotype 1, however intravenous administration using other serotypes may also be applicable (AAV6, AAV8).

General methods for gene therapy are known in the art. See for example, U.S. Pat. No. 5,399,346 by Anderson et al*.* (incorporated herein by reference). A biocompatible capsule for delivering genetic material is described in PCT Publication WO 95/05452 by Baetge et al*.* Methods of gene transfer into hematopoietic cells have also previously been reported (see Clapp, D. W., et al., Blood 78: 1132-1139 (1991); Anderson, Science 288:627-9 (2000); and , Cavazzana-Calvo et al., Science 288:669-72 (2000).

### Effective amounts

In the treatment according to the invention, non-alcoholic steatotic hepatitis is treated by administering to a subject an effective amount of an LPL therapeutic. As used herein, non-alcoholic steatotic hepatitis refers to the development of histological changes in the liver that are comparable to those induced by excessive alcohol intake, but in the absence of alcohol abuse.

The LPL therapeutic will typically be included in a pharmaceutical composition, optionally in combination with a pharmaceutical carrier, diluent and/or adjuvant. Such compositions include the LPL therapeutic in an effective amount, sufficient to provide a desired therapeutic or prophylactic effect, and a pharmaceutically acceptable carrier or excipient. An "effective amount" includes a therapeutically effective amount or a prophylactically effective amount.

A "therapeutically effective amount" refers to an amount effective, at dosages and for periods of time necessary, to achieve the desired therapeutic result, such as alteration of parameters in lipid metabolism, such as elevation of LPL activity, elevation of HDL-cholesterol or reduction of triglyceride levels. A therapeutically effective amount of an LPL therapeutic may vary according to factors such as the disease state, age, sex, and weight of the individual, and the ability of the LPL therapeutic to elicit a desired response in the individual. Dosage regimens may be adjusted to provide the optimum therapeutic response. A therapeutically effective amount is also typically one in which any toxic or detrimental effects of the LPL therapeutic are outweighed by the therapeutically beneficial effects.

A "prophylactically effective amount" refers to an amount effective, at dosages and for periods of time necessary, to achieve the desired prophylactic result, such as preventing or inhibiting various conditions, including LPL responsive conditions, such as coronary heart disease, cardiovascular disease, coronary artery disease, high triglycerides and/or low HDL. A prophylactic dose may be used in subjects prior to or at an earlier stage of disease, and a prophylactically effective amount may be more or less than a therapeutically effective amount in some cases.

In particular embodiments, a range for therapeutically or prophylactically effective amounts of LPL therapeutic may be 0.01 nM-0.1M, 0.1 nM-0.1M, 0.1 nM-0.05M, 0.05 nM-15µM or 0.01 nM-10µM. It is to be noted that dosage values may vary with the severity of the condition to be alleviated. For any particular subject, specific dosage regimens may be adjusted over time according to the individual need and the professional judgement of the person administering or supervising the administration of the compositions. Dosage ranges set forth herein are exemplary only and do not limit the dosage ranges that may be selected by medical practitioners.

For gene therapy vectors, the dosage to be administered may depend to a large extent on the condition and size of the subject being treated as well as the therapeutic formulation, frequency of treatment and the route of administration. Regimens for continuing therapy, including dose, formulation, and frequency may be guided by the initial response and clinical judgment. The parenteral route of injection into the interstitial space of tissue may be preferred, although other parenteral routes, such as inhalation of an aerosol formulation, may be required in specific administration. In some protocols, a formulation comprising the gene and gene delivery system in an aqueous carrier is injected into tissue in appropriate amounts. The tissue target may be specific, for example the muscle or liver tissue, or it may be a combination of several tissues, for example the muscle and liver tissues. Exemplary tissue targets may include liver, skeletal muscle, heart muscle, adipose deposits, kidney, lung, vascular endothelium, epithelial and/or hematopoietic cells.

In one embodiment, the effective dose range for small animals (mice), following intramuscular injection, is between 1x10¹² and 1x10¹³ genome copy (gc)/kg, and for larger animals (cats) and possibly human subjects, between 1x10¹¹ and 1x10¹² gc/kg. Again in mice, levels of transgene expression, as measured by LPL in post-heparin plasma, reach up to 300 ng/ml (measured using a commercial ELISA from DaiNippon), and expression is long-term (>1 year) following a one-time administration.

The amount of active compound in the compositions of the invention may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage. "Dosage unit form" as used herein refers to physically discrete units suited as unitary dosages for subjects to be treated; each unit containing a predetermined quantity of active compound calculated to produce the desired therapeutic effect in association with the required pharmaceutical carrier. The specification for the dosage unit forms of the invention may be dictated by the unique characteristics of the active compound and the particular therapeutic effect to be achieved, and by the limitations inherent in the art of compounding such an active compound for the treatment of a condition in individuals.

As used herein "pharmaceutically acceptable carrier" or "exipient" includes any and all solvents, dispersion media, coatings, antibacterial and antifungal agents, isotonic and absorption delaying agents, and the like that are physiologically compatible. In one embodiment, the carrier is suitable for parenteral administration, whichincludes intravenous, intraperitoneal or intramuscular administration. Alternatively, the carrier may be suitable for sublingual or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated.

Supplementary active compounds can also be incorporated into the pharmaceutical compositions of the invention. Guidance on co-administration of additional therapeutics may for example be found in the Compendium of Pharmaceutical and Specialties (CPS) of the Canadian Pharmacists Association.

Pharmaceutical compositions are typically sterile and stable under the conditions of manufacture and storage. Pharmaceutical compositions may be formulated as a solution, microemulsion, liposome, or other ordered structure suitable to accommodate high drug concentration. The carrier may be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin. The LPL therapeutic may be administered in a time or controlled release formulation, for example in a composition which includes a slow release polymer or other carriers that will protect the compound against rapid release, including implants and microencapsulated delivery systems. Biodegradable, biocompatible polymers may for example be used, such as ethylene vinyl acetate, polyanhydrides, polyglycolic acid, collagen, polyorthoesters, polylactic acid and polylactic, polyglycolic copolymers (PLG). Many methods for the preparation of such formulations are patented or generally known to those skilled in the art.

### Short description of the figures

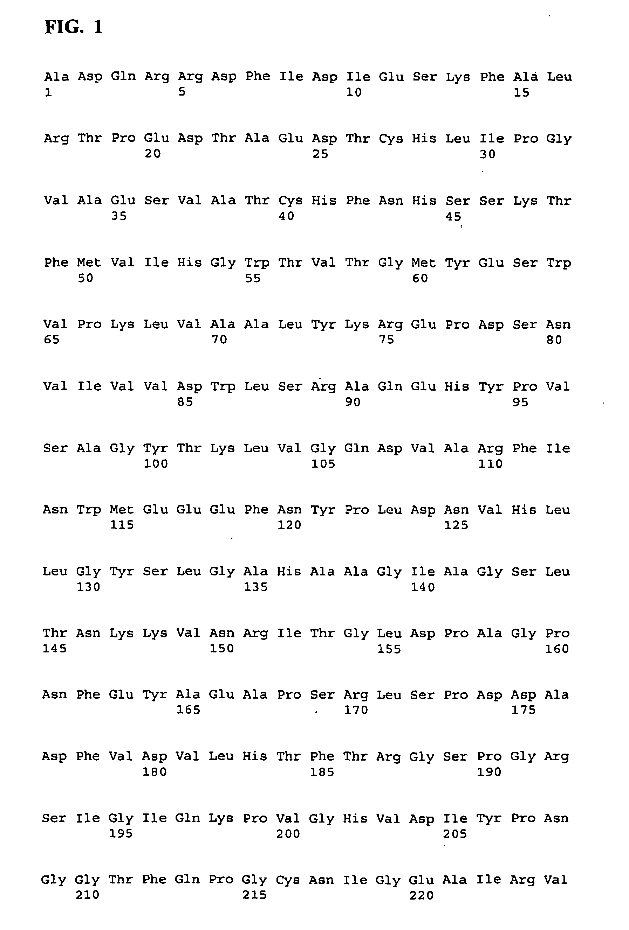

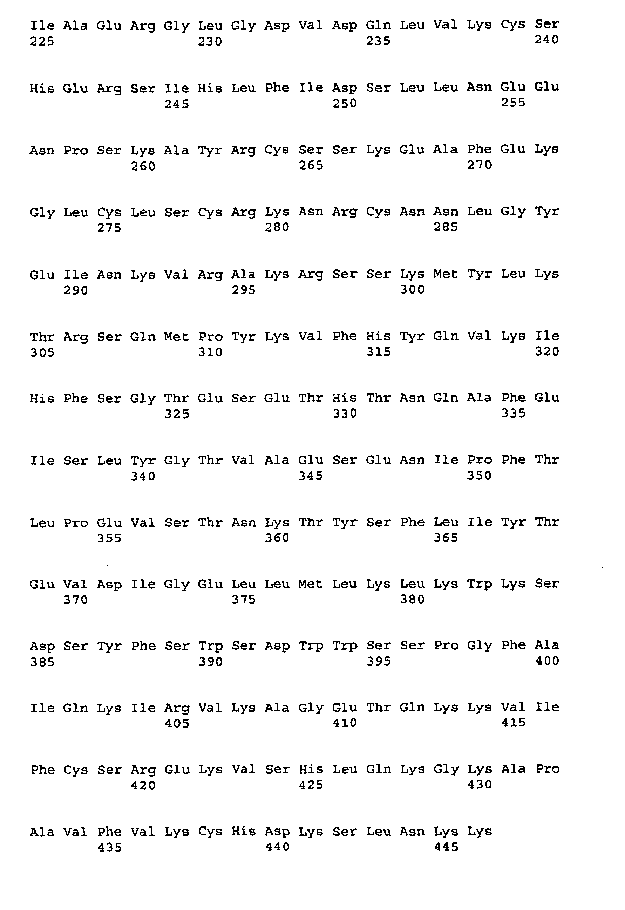

- Figure 1:: Protein sequence of one embodiment of an LPL therapeutic according to the invention, i.e. an S447X protein.

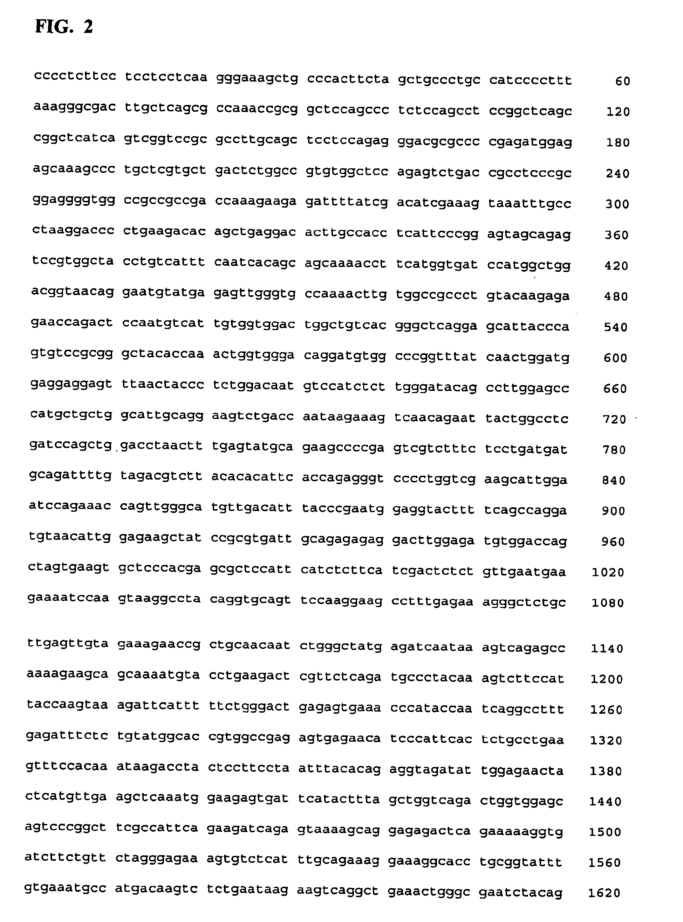

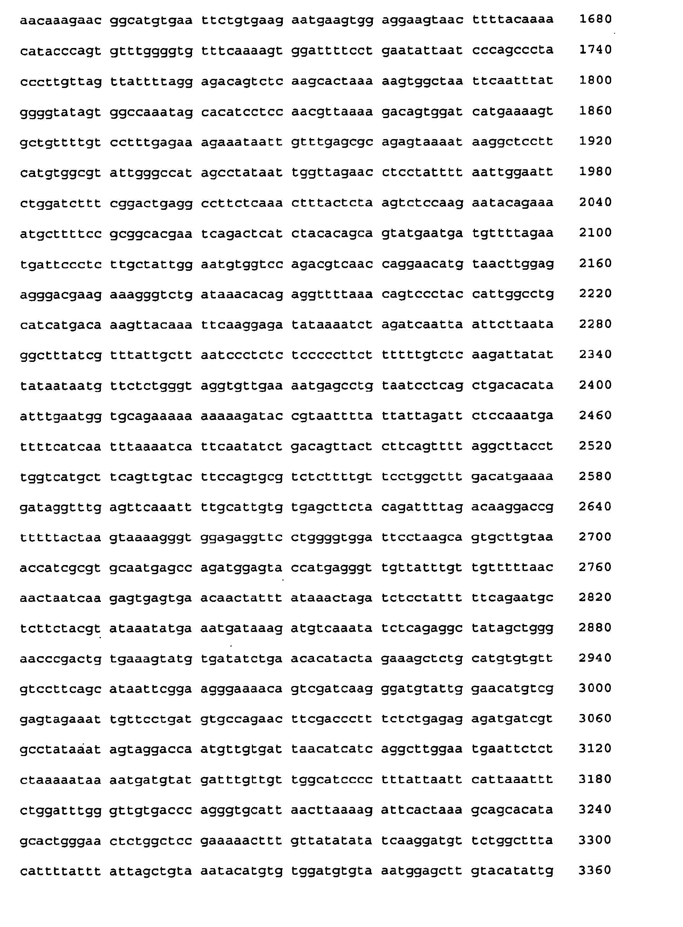

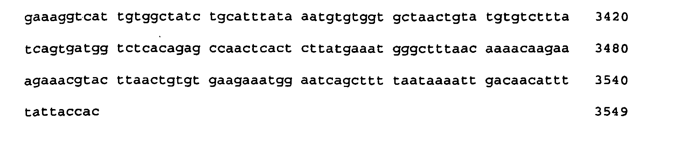

- Figure 2:: LPL mRNA sequence. Nucleotides 256 through 1599 encode the mature wild type LPL peptide.

### EXAMPLE

### Example 1 LPL gene therapy causes triglyceride redistribution

This example investigates the effects of AAV1 with LPL S447X as transgene on diet-induced hyperlipidemia in male APOE3-Leiden transgenic mice. For this purpose we injected 6 mice with 1x10¹³ gc's/kg AAV1- LPL S447X and 6 mice with PBS (intramuscular; 4 sites). One week later the mice were started on a Western type diet. At 28 weeks after starting the diet, the mice were sacrificed.

The results show that mice that received LPL gene therapy showed a gradual increase in post-heparin LPL concentrations up to 300 ng/ml at 25 weeks after injection, but total post-heparin lipase activity was not significantly changed. In addition, no effects on plasma TG, HDL-c and TC after 4 hrs fasting could be detected. Despite this absence of an effect on circulating lipoprotein concentrations, LPL gene therapy proved effective in that the treated mice displayed a faster clearance of TG and FFA after a bolus of intravenously administered Intralipid (p<0.05). Other significant findings included a significantly reduced gain of weight of the AAV1- LPL S447X treated animals compared to the controls (p<0.05), an effect that could not be attributed to a difference in abdominal fat mass. Muscle homogenates of the AAV1- S447X treated mice indicated significantly increased triglyceride content (p<0.05) while liver homogenates indicated significantly decreased triglyceride content (p<0.05) compared to controls.

This example demonstrates that in the absence of an effect on fasting lipids, expression of human LPL in the mouse skeletal muscle induces a redistribution of triglycerides and a reduced gain of body weight.

**Table 1**

| **Human LPL concentration post heparin plasma after AAV1-LPLS447X injection** | | | | |
|---|---|---|---|---|
| | Week 0 | Week 1 | Week 11 | Week 25 |
| Mean (ng/ml) | 0 | 107 | 187 | 293 |
| Stdev | 0 | 42 | 59 | 250 |

**Table 2**

| **Fat tolerance test of peak triglycerides, % of value after injection of intra lipid** | | | | | |
|---|---|---|---|---|---|
| Time after injection intra lipid (min.) | Mean PBS group | STdev | Mean AAV1-LPL | STdev | P (T-test) |
| <3 | 100.0 | 0.0 | 100.0 | 0.0 | |
| 30 | 40.7 | 13.1 | 21.2 | 4.7 | <0.05 |
| 60 | 9.3 | 5.3 | 5.1 | 2.3 | |
| 120 | 3.6 | 1.1 | 2.5 | 0.9 | |

**Table 3**

| **Fat tolerance test of peak free fatty acids, % of value after injection of intra lipid** | | | | | |
|---|---|---|---|---|---|
| Time after injection intra lipid (min.) | Mean PBS group | Stdev | Mean AAV1-LPL | Stdev | P (T-test) |
| <3 | 100.0 | 0.0 | 100.0 | 0.0 | |
| 30 | 56.9 | 13.7 | 33.8 | 6.4 | <0.05 |
| 60 | 14.0 | 5.9 | 12.8 | 4.6 | |
| 120 | 9.3 | 2.8 | 11.5 | 3.1 | |

## Claims

1. The use of a lipoprotein lipase (LPL) therapeutic for the preparation of a medicament for the treatment of non-alcoholic steatotic hepatitis in a subject.

2. The use according to claim 1 wherein the LPL therapeutic is a member selected from the group consisting of:
a) an S447X protein with an amino acid sequence as shown in SEQ ID No. 1, or a derivative thereof;
b) an LPL protein with an amino acid sequence which comprises a contiguous segment having at least 90% sequence identity to SEQ ID NO:1 when optimally aligned which has equal or greater LPL activity than the protein under a), or a derivative thereof; or
c) a nucleic acid encoding a) or b), or a derivative thereof.

3. The use according to claim 1 or 2, wherein the LPL therapeutic comprises a nucleic acid which comprises a DNA coding sequence encoding an RNA having at least 90% sequence identity to nucleotides 256 through 1599 of SEQ ID NO: 2.

4. The use according to claims 1-3, wherein the LPL therapeutic is a nucleic acid which comprises a DNA coding sequence that hybridizes under stringent conditions to nucleotides 256 through 1599 of SEQ ID NO:2.

5. The use according to claim 1 or 2, wherein the LPL therapeutic is an LPL S447X protein with a contiguous segment of at least 95% sequence identity to SEQ ID NO:1.

6. The use according to claims 1-5, wherein the LPL therapeutic is to be administered to the subject in a gene therapy vector.

7. The use according to claim 6, wherein the gene therapy vector comprises a viral vector.

8. The use according to claim 7, wherein the viral vector comprises adeno-associated virus (AAV).

9. The use according to claims 1-8, wherein the subject is a human.

10. The use according to claims 1-9, wherein the LPL therapeutic is to be administered parenterally.

## Patentansprüche

1. Verwendung eines Lipoprotein-Lipase (LPL)-Therapeutikums für die Herstellung eines Medikaments zur Behandlung einer nichtalkoholischen Steatohepatitis in einem Individuum.

2. Verwendung nach Anspruch 1, wobei das LPL-Therapeutikum ein Mitglied ist, ausgewählt aus der Gruppe bestehend aus:
(a) einem S447X-Protein mit einer Aminosäuresequenz wie in SEQ ID NO: 1 gezeigt oder einem Derivat davon;
(b) einem LPL-Protein mit einer Aminosäuresequenz, die ein zusammenhängendes Segment umfasst, das bei optimaler Ausrichtung mindestens 90% Sequenzidentität Zu SEQ ID NO: 1 aufweist und eine gleich große oder größere LPL-Aktivität als das Protein aus (a) aufweist, oder einem Derivat davon; oder
(c) einer Nucleinsäure, die (a) oder (b) codiert, oder einem Derivat davon.

3. Verwendung nach Anspruch 1 oder 2, wobei das LPL-Therapeutikum eine Nucleinsäure umfasst, die eine DNA-codierende Sequenz umfasst, die eine RNA mit mindestens 90% Sequenzidentität zu den Nucleotiden 256 bis 1599 der SEQ ID NO: 2 codiert.

4. Verwendung nach Anspruch 1 bis 3, wobei das LPL-Therapeutikum eine Nucleinsäure ist, die eine DNA-codierende Sequenz umfasst, die unter stringenten Bedingungen an die Nucleotide 256 bis 1599 der SEQ ID NO: 2 hybridisiert.

5. Verwendung nach Anspruch 1 oder 2, wobei das LPL-Therapeutikum ein LPL S447X-Protein mit einem zusammenhängenden Segment von mindestens 95% Sequenzidentität zu SEQ ID NO: 1 ist.

6. Verwendung nach Anspruch 1 bis 5, wobei das LPL-Therapeutikum dem Individuum in einem Gentherapievektor zu verabreichen ist.

7. Verwendung nach Anspruch 6, wobei der Gentherapievektor einen viralen Vektor umfasst.

8. Verwendung nach Anspruch 7, wobei der virale Vektor ein Adeno-assoziiertes Virus (AAV) umfasst.

9. Verwendung nach Anspruch 1 bis 8, wobei das Individuum ein Mensch ist.

10. Verwendung nach Anspruch 1 bis 9, wobei das LPL-Therapeutikum parenteral zu verabreichen ist.

## Revendications

1. Utilisation d'une lipoprotéine lipase (LPL) thérapeutique pour la préparation d'un médicament destiné au traitement d'une hépatite stéatosique non alcoolique chez un sujet.

2. Utilisation selon la revendication 1, la LPL thérapeutique étant choisie dans le groupe constitué :
a) d'une protéine S447X dont la séquence d'acides aminés est telle que présentée dans SEQ ID NO : 1, ou d'un dérivé de celle-ci ;
b) d'une protéine LPL dont la séquence d'acides aminés comprend un segment contigu présentant au moins 90 % d'identité de séquence avec SEQ ID NO : 1 en cas d'alignement optimal et qui présente une activité LPL égale ou supérieure à celle de la protéine du paragraphe a), ou d'un dérivé de celle-ci ; ou
c) d'un acide nucléique codant pour la protéine du paragraphe a) ou b), ou d'un dérivé de celui-ci.

3. Utilisation selon la revendication 1 ou 2, la LPL thérapeutique comprenant un acide nucléique comportant une séquence d'ADN codante encodant un ARN présentant au moins 90 % d'identité de séquence avec les nucléotides 256 à 1 599 de SEQ ID NO : 2.

4. Utilisation selon les revendications 1 à 3, la LPL thérapeutique étant un acide nucléique comprenant une séquence d'ADN codante qui s'hybride dans des conditions stringentes avec les nucléotides 256 à 1 599 de SEQ ID NO : 2.

5. Utilisation selon la revendication 1 ou 2, la LPL thérapeutique étant une protéine LPL de type S447X avec un segment contigu présentant au moins 95 % d'identité de séquence avec SEQ ID NO : 1.

6. Utilisation selon les revendications 1 à 5, la LPL thérapeutique étant destinée à être administrée au sujet dans un vecteur de thérapie génique.

7. Utilisation selon la revendication 6, le vecteur de thérapie génique comprenant un vecteur viral.

8. Utilisation selon la revendication 7, le vecteur viral comprenant un virus adéno-associé (AAV).

9. Utilisation selon les revendications 1 à 8, le sujet étant un être humain.

10. Utilisation selon les revendications 1 à 9, la LPL thérapeutique étant destinée à être administrée par voie parentérale.
